(19) Europäisches Patentamt

European Patent Office

Office européen des brevets

(11) Veröffentlichungsnummer: **0 461 505 B1**

(12) **EUROPÄISCHE PATENTSCHRIFT**

(45) Veröffentlichungstag der Patentschrift: **22.02.95**

(51) Int. Cl.⁶: **A61K 31/21**, A61K 9/72

(21) Anmeldenummer: **91109019.9**

(22) Anmeldetag: **03.06.91**

(54) **Nitroglycerin-Spray.**

(30) Priorität: **13.06.90 DE 4018919**

(43) Veröffentlichungstag der Anmeldung:
**18.12.91 Patentblatt 91/51**

(45) Bekanntmachung des Hinweises auf die
Patenterteilung:
**22.02.95 Patentblatt 95/08**

(84) Benannte Vertragsstaaten:
**AT BE CH DE DK ES FR GB GR IT LI LU NL SE**

(56) Entgegenhaltungen:
**US-A- 3 155 574**

**J. OF PHARM. SC., Band 63, Nr. 6, Juni 1974, Seiten 907-911; A.M. CONTRACTOR etal.: "Development and evaluation of an inhalation aerosol of nitroglycerin"**

**IDEM**

**JAHRESTAG. INST. CHEM. TREIB-EXPLOSIV-STOFFE FRAUNHOFER-GES. 1971 (Publ. 1972),Seiten 375-399, Inst. Chem. Treib.-Explosivstoffe, Berghausen, DE; R. STENSON:**

**"The interactions of cellulose acetate and**

**ethyl cellulose inhibitors withdouble-base propellants"**

**IDEM**

(73) Patentinhaber: **SCHWARZ PHARMA AG
Alfred-Nobel-Strasse 10
D-40789 Monheim (DE)**

(72) Erfinder: **Klokkers-Bethke, Karin, Dr.
Görlitzer Strasse 16
W-6074 Rödermark (DE)**
Erfinder: **Münch, Ulrich, Dr.
Am Wald 34
W-4019 Monheim/Rhld (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft einen Nitroglycerin-Spray auf hydrophiler Basis mit definierter Zusammensetzung und in ständigem Kontakt mit der Sprayzusammensetzung stehendem Dichtungsmaterial, dessen Absorptionswert für Nitroglycerin geringer als 10 mg/1 g Dichtungsmaterial ist.

Nitroglycerin, auch als GTN bezeichnet, ist ein Wirkstoff zur Behandlung von Angina pectoris-Anfallen. Er wird u.a. in Notfallsituationen eingesetzt, in denen die Arzneiform einen schnellen Wirkungseintritt ermöglichen sollte.

Die in dieser speziellen Indikation eingesetzten Arzneiformen wie Sublingualtabletten oder Zerbeißkapseln besitzen Nachteile. Nachteilig ist u.a., daß in diesen Arzneiformen der Wirkstoff nach der Einnahme erst freigesetzt und verteilt werden muß, bevor er gelöst zur Resorption zur Verfügung steht. Weiterhin tritt unnötiger Zeitverlust bei der akuten Anfallsbehandlung ein, wenn die Arzneiform erst aus Packung und Blister entnommen werden muß.

Zur Vermeidung der Nachteile dieser Arzneiformen wurden GTN-haltige Sprays entwickelt. Durch Aufsprühen der wirkstoffhaltigen Dosis in den Mund sollte ein direktes und schnelles Aufbringen einer Lösung des Wirkstoffes auf einen möglichst großen Teil der den Wirkstoff GTN gut resorbierenden Mundschleimhaut gewährleistet werden. Hierdurch soll eine große Fläche erreicht werden, die die Diffusion des Wirkstoffes beschleunigt.

Allerdings setzen die Eigenschaften des GTN wie hoher Dampfdruck, das Lösungsverhalten und die Explosivität diesem Ziel bisher Grenzen. Aus Sicherheitsgründen müssen daher bei der Formulierung der Arzneiform Spray phlegmatisierte Wirkstoff-Hilfsstoff-Mischungeneingesetzt werden.

Aufgrund seiner Lipophilie ist GTN gut löslich in Lösemitteln wie Ether, Aceton, Ethylacetat, Benzol, Chloroform und Triglyceriden. Dagegen ist die Löslichkeit in hydrophilen Lösemitteln wie Wasser begrenzt. Die Löslichkeit in Wasser beträgt lediglich etwa 1,1 mg/ml GTN.

Demgemäß sind die in herkömmlichen Sprayformulierungen verwendeten Lösungsmittel lipophil. Üblich sind Öle oder Triglyceride.

Allerdings verhindern lipophile Lösungsmittel, daß der Wirkstoff GTN mit der bei Akutanfällen der Angina pectoris erwünschten Schnelligkeit in die hydrophile Mucosa verteilt wird.

In der Vergangenheit war ein Ansatzpunkt zur Erhöhung der Wirkstoffverfügbarkeit, daß die Menge der eingesetzten lipophilen Lösungsmittel vermindert wurde. Jedoch wurde die Anflutdauer, meßbar über die maximale Plasmakonzentration ($C_{max}$) und Zeitpunkt der maximalen Konzentration ($t_{max}$), nur geringfügig beeinflußt.

P.M. Dewland et al. [in Herz & Gefäße, 7, 536-544 ( 1987)] erhielten zwar für drei mit lipophilen Lösungen hergestellte GTN-Sprays mit abnehmender Menge der lipophilen Rezepturanteile höhere $C_{max}$-Werte (Tab. 1), jedoch ist $t_{max}$ nicht signifikant unterschiedlich.

Ein anderer Ansatzpunkt, die Verfügbarkeit des Wirkstoffes zu erhöhen, wird in DE-A 32 46 081 beschrieben.

Dies wird dadurch erreicht, daß der Treibgasanteil auf 60-95 % der Rezepturbestandteile erhöht wird.

Der erhöhte Treibgasanteil bewirkt hierbei eine höhere Konzentration des Wirkstoffes im nichtflüchtigen, öligen Lösemittel. Der Arzneistoff muß weiterhin erst aus der öligen Wirkstofflösung in die Mucosa diffundieren. Die im Anfallgeschehen wichtige Anflutung des Wirkstoffes kann dadurch jedoch nicht bedeutend verkürzt werden. Auch aus Gesichtspunkten gesteigerten Umweltbewußtseins ist ein erhöhter Treibgasanteil nachteilig und daher zu vermeiden.

Eine qualitativ deutliche Verbesserung des Sprays im Anfallsgeschehen ist unter Beibehaltung lipophiler Lösemittel nicht möglich.

Ein weiterer Ansatzpunkt, den Wirkstoff schnell in die hydrophile Mucosa zu verteilen, ist die Verwendung eines Lösemittels, dessen Löseeigenschaften für den Wirkstoff GTN möglichst gering sind. Die Sprayformulierung muß hierbei berücksichtigen, daß dieses Lösemittel oder Lösemittelgemisch den Wirkstoff GTN ausreichend phlegmatisiert und auch technisch unter Produktionsbedingungen sicher handzuhaben ist.

US-PS 3,155,574 beschreibt eine Nitroglycerinsprayformulierung zur Inhalation auf hydrophiler Lösemittelbasis, enthaltend den Wirkstoff GTN, 1,2-Propandiol und wasserfreies Ethanol, praktische Ausführungsbeispiele der Primärpackmittel fehlen. Die Inhalation ist jedoch im Anfallsgeschehen eher von Nachteil, da sie schwieriger durchzuführen ist. Wünschenswert sind GTN-Sprays, bei denen der Wirkstoff ausreichend über die orale Mucosa absorbiert wird und sich demgemäß die Inhalation des Wirkstoffes erübrigt.

Untersuchungen von H. Laufen et al. in Therapiewoche 34, 963-970 (1984) brachten das Ergebnis, daß im Falle der hydrophilen Rezeptur im Vergleich zu GTN-Sprays auf lipophiler Basis sowohl das Erscheinen des Wirkstoffes im Blut als auch die Menge der resorbierten Substanz schneller bzw. größer ist. Die Autoren benutzen zur Dosierung der Lösung einen Pumpspray. Nach der allgemeinen Erfahrung erfüllen Pumpsprays bisher nicht die an Arzneimittel gestellten Anforderungen, so daß die Formulierung mit dem therapeutisch günstigen Effekt nicht in ein Arzneimittel umgesetzt werden kann.

EP-A 0 310 910 beschreibt eine Nitroglycerin-Spray-Formulierung, die außer dem Wirkstoff lediglich Ethanol und Wasser als Lösemittel enthält und auf einen pH-Bereich von 2,4 bis 6,7 eingestellt ist. Allerdings erfährt in dieser Formulierung der Wirkstoff bei Verdampfen des Ethanols eine Phasentrennung aus dem Wasser und liegt somit nicht phlegmatisiert vor, wogegen gerade die Phlegmatisierung aus Sicherheitsgründen wünschenswert wäre.

Der bisherige Stand der Technik von Sprays auf hydrophiler Basis im Vergleich zu denen auf lipophiler Basis zeigt Mängel wie eine Absorption von Nitroglycerin in Ventilbestandteile und eine Verringerung der Dosismenge Wirkstoff GTN bei jedem neuen Sprühstoß.

Überraschenderweise wurde nun gefunden, daß ein Nitroglycerinspray auf hydrophiler Basis in einer speziellen Zusammensetzung, die in Verbindung mit Behältnis-Dichtmaterial, dessen Absorptionswert für Glyceroltrinitrat geringer als 10 mg/1 g Dichtmaterial beträgt, einen konstanten Wirkstoffgehalt pro Einzeldosierung aufweist.

Gegenstand der Erfindung ist dementsprechend ein Nitroglycerin-Spray, der aus den Bestandteilen Glyceroltrinitrat, Ethanol, 1,2-Propylenglykol, Dichlordifluormethan und Dichlortetrafluorethan besteht, die in einem bestimmten Gewichtsverhältnis zueinander stehen und sich in ständigem Kontakt mit Behältnisdichtmaterial aus Kunststoff befinden, dessen Absorptionswert für GTN geringer als 10 mg/1 g Dichtmaterial ist.

Das erfindungsgemäße Nitroglycerin-Spray ist ein Nitroglycerin-Dosier-Aerosol, das sich aus 0,73 Gew.-% Nitroglycerin in einer hydrophilen Lösung aus 13,83 Gew.-% Ethanol und 7,28 Gew.-% 1,2-Propylenglykol sowie 31,26 Gew.-% Dichlordifluormethan und 46,90 Gew.-% Dichlortetrafluorethan zusammensetzt, diese Zusammensetzung in ständigem Kontakt mit dem Behältnisdichtmaterial steht, dessen Absorptionswert für GTN geringer als 10 mg/1 g Dichtmaterial ist.

Nach einer erfindungsgemäßen Ausführungsform ist das Behältnisdichtmaterial Butyl FA 7500.

Eine weitere Ausführungsform enthält kohlenstofffreies Neopren als Behältnisdichtmaterial.

Erfindungsgemäß findet in treibgashaltigen Nitroglycerin-Sprayvorrichtungen Behältnisdichtmaterial mit Absorptionswerten für Nitroglycerin unter 10 mg/1 g Dichtmaterial Verwendung.

Außer den zuvor genannten Hauptbestandteilen kann der erfindungsgemäße Nitroglycerin-Spray übliche Zusätze wie beispielsweise Geschmacksstoffe enthalten, die dem Fachmann wohlbekannt sind.

Die Herstellung des erfindungsgemäßen Nitroglycerinsprays erfolgt in der Weise, daß eine homogene, einphasige Lösung aus Glyceroltrinitrat, Ethanol und 1,2-Propylenglykol, wie in den in der Tabelle 1 angegebenen Gewichts-%, in Aerosoldosen oder -flaschen gefüllt, ein geeignetes Dosierventil, dessen Absorptionswert für Glyceroltrinitrat geringer als 10 mg/1g Dichtmaterial beträgt, aufgecrimpt und mit einer fertigen Mischung aus Dichlordifluormethan und Dichlortetrafluorethan, wie in den in der Tabelle 1 angegebenen Gewichts-% begast wird.

<u>Tabelle 2</u> gibt einen vergleichenden Überblick über die Rezepturzusammensetzung des Standes der Technik und des erfindungsgemäßen Sprays.

Die Zusammensetzung eines erfindungsgemäßen Sprays lautet:

| <u>lfd. Nr.</u> | <u>Bezeichnung</u> | <u>in 100 g</u> |
|---|---|---|
| 1 | Glyceroltrinitrat | 0,73 |
| 2 | Ethanol | 13,83 |
| 3 | 1,2-Propylenglykol | 7,28 |
| 4 | Dichlordifluormethan | 31,26 |
| 5 | Dichlortetrafluorethan | 46,90 |
| 6 | Dichtmaterial mit einem Absorptionswert für GTN unter 10 mg/1 g Dichtmaterial | |

Die hydrophile Sprayzusammensetzung ist im Fertigprodukt in Kontakt mit dem Behältermaterial und den Dosierventilmaterialien.

Stabilitätsuntersuchungen an Sprays mit identischem erfindungsgemäßem Inhalt, identischen Behältern, aber qualitativ unterschiedlichen Ventilen zeigten, daß die pharmazeutische Qualität des Produktes nur bei Verwendung von Ventilen, hergestellt mit speziellen Materialien, gewährleistet ist. Nur bei diesen speziellen Materialien findet keine oder eine tolerierbare Absorption des Wirkstoffes Glyceroltrinitrat in die Ventilbestandteile statt.

In Tab. 3 ist die Wechselwirkung der kritischen Ventilbestandteile, dies sind Dichtungen, mit dem erfindungsgemäßen Spray dargestellt. Die Wirkstoffabsorption in die Dichtungen wurde bestimmt durch Quantifizierung des Glyceroltrinitrates nach Extraktion aus Ventildichtungen nach 2- bzw. 4-wöchiger Inkubation im Spray.

Die WE-Nummer ist eine Identitätsnummer des Ventils, aus dem das jeweilige Dichtungsmaterial entnommen wurde. Bei der Herstellung von erfindungsgemäßem Spray wurde zusätzlich zur Wirkstofflösung eine definierte Masse Dichtmaterial in den Spraybehälter gegeben, ein Ventil aufgecrimpt, begast und das Spray aufrecht bei der entsprechenden Bedingung gelagert. Zum Untersuchungstermin wurde die Sprayflasche im gekühlten Zustand entgast, die Dichtungen entnommen, gewaschen und der GTN-Gehalt nach Extraktion bestimmt.

Die Prüfung ergab als Resultat die in Tab. 3 aufgelisteten Werte.

Die Glyceroltrinitrat-Absorption bei 20 °C beträgt 53 mg GTN/1 g Dichtung Nr. 400, 45 mg GTN/1 g Buna BA 170 T, 3,4 mg GTN/1 g Neopren beige und ≤ 1 mg GTN/1 g Butyl FA 7500.

Ventile mit Dichtungsmaterialien eines Absorptionswertes von > 10 mg/1 g Dichtungsmaterial führen während Lagerung des Sprays zur Wirkstoffverminderung in der Packung, so daß der Wirkstoffgehalt pro Einzeldosierung unter die arzneimittelrechtlich akzeptierten Limits fällt. Im weiteren führen Ventile mit diesen stark absorbierenden Dichtungsmaterialien, die im Bereich der Dosierkammer Kontakt zur nächsten zu applizierenden Dosis haben, dort ebenfalls zu nicht mehr zulässigen Mindergehalten.

Tab. 4 und 5 zeigen dieses Phänomen an vier Chargen von Sprays auf, die mit unterschiedliche Dichtungsmaterialien enthaltenden Ventilen hergestellt wurden. Dargestellt ist die gefundene Wirkstoffmenge in % der Solldosis im 1., 2. und 3. Einzelsprühstoß nach unterschiedlichen, benutzungsfreien Zeitintervallen. Nur Ventile, die Butyl FA 7500 und Neopren enthalten, gewährleisten eine ausreichende pharmazeutische Qualität.

Die praktische Ausführung des Arzneimittels erfordert somit die Verwendung von Dosierventilen mit speziellen Dichtungsmaterialien.

Hierfür ist der unterschiedliche Verteilungskoeffizient (Pu) des GTN zwischen den Bestandteilen der Rezeptur (R) und der hydrophilen Mundschleimhaut (M) bestimmend:

$P_u = \frac{M}{R}$ ist im Falle der hydrophilen Rezeptur größer als im Falle der lipophilen, wodurch die schnelle Anflutung in vivo erreicht wird. Die Verteilung $P_k = \frac{D}{R}$ (Dichtung = D), d.h. in identische Dichtungen ist aber dann auch größer im Falle des hydrophilen Sprays und macht den Einsatz von neuen, speziellen Dichtungsmaterialien erforderlich, die eine geringe Löslichkeit für GTN bzw. eine geringere Absorption aufweisen.

Mit dem erfindungsgemäßen Glyceroltrinitrat Dosierspray wurde in einer Bioverfügbarkeitsstudie und in einer klinischen Studie das beabsichtigte schnellere Anfluten des Wirkstoffes im Vergleich zu langsameren lipophilen Sprays belegt und die schnelle Wirkung gezeigt.

Therapeutisch von Bedeutung ist eine schnelle Anflutung ($t_{max}$) zu hohen Wirkstoffkonzentrationen. Dies führt zu einem schnellen Wirkungseintritt und zu einer effektiven Wirkung. Damit verbunden ist eine frühere Kupierung des Angina pectoris-Anfalles und eine schnelle Hilfe für den Patienten in dieser von Todesangst geprägten, lebensbedrohlichen Situation.

Zwei galenisch verschiedene GTN-Sprays wurden bei identischer Applikationsart miteinander verglichen.

Der Testspray (T) ist der erfindungsgemäße Spray mit dem besonderen Dichtungsmaterial, der den Wirkstoff in einem hydrophilen Lösungsmittel enthält. Der Referenzspray (R) enthält den Wirkstoff in einem lipophilen Lösungsmittel.

Das Prüfungsergebnis zeigt Tabelle 6.

Tabelle 6

| Parameter | T | R | T/R | P |
|---|---|---|---|---|
| $C_{max}$ [pg/ml] | 1774 ± 1272 | 884 ± 693 | 2.16 | 0.006 |
| $t_{max}$ [min] | 4.4 ± 1.4 | 7.9 ± 2.8 | | < 0.05 |
| AUC [pg min/ml] | 9488 ± 5303 | 6990 ± 5168 | 1.52 | n.s. |

Die Prüfung zeigt, daß der Wirkstoff aus dem Testpräparat signifikant schneller absorbiert wird als aus dem Referenzspray; $C_{max}$ war um den Faktor 2.2 höher und $t_{max}$ von 7.9 auf 4.4 min verkürzt.

Der erfindungsgemäße Spray bewirkt das schnellere Anfluten des Wirkstoffes bei guter Qualität des pharmazeutischen Produktes. Für den Angina pectoris-Patienten im Anfallsgeschehen bedeutet das eine schnellere Wirkung und eine schnellere Beseitigung der im Anfallsgeschehen auftretenden Angstzustände.

EP 0 461 505 B1

Tab. 1 Literaturdaten pharmakokinetischer Parameter zu GTN – Spray bei sublingualer Applikation

| Präparat | Quelle Nr. | Probanden (Anzahl) | Dosis | $C_{max}$ (pg/ml) | $t_{max}$ (min) | AUC (min $\cdot$ pg/ml$^{-1}$) | rel.AUC ( % ) |
|---|---|---|---|---|---|---|---|
| lipophiler Spray A | 1 | 3 | 0,4 | 1. 400<br>2. 860<br>3. 590 | 4-5<br>3<br>5 | – | |
| lipophiler Spray B | 1 | 3 | 0,4 | 1. 2260<br>2. 1620<br>3. 1444 | 3,2<br>6,5<br>10,0 | – | |
| lipophiler Spray C | 2 | 12 | 0,8 | 670 ± 500 | 10,0 | 5740 ± 4590 | 56,26 |
| lipophiler Spray D | 2 | 12 | 0,8 | 760 ± 450 | 8,0 | 6360 ± 3970 | 58,9 |
| lipophiler Spray B | 2 | 12 | 0,8 | 1150 ± 770<br>p < 0,05 | 8,0<br>p >0,05 | 9990 ± 8080<br>p < 0,05 | 100 |
| lipophiler Spray A | 3 | 8 | 0,8 | 780 ± 850 | 7 | 1369 ± 16040 | 36,5 |
| hydrophiler Spray A | 3 | 8 | 0,8 | 3810 ± 2810 | 4 | 37460 ± 14640<br>p < 0,05 | 100 |

Fortsetzung Tab. 1

| Präparat | Quelle Nr. | Probanden (Anzahl) | Dosis | $C_{max}$ (pg/ml) | $t_{max}$ (min) | AUC (min $\cdot$ pg/ml$^{-1}$) | rel.AUC ( % ) |
|---|---|---|---|---|---|---|---|
| lipophiler Spray B | 4 | 12 | 0,8 | 340 ± 234 | 8,3 ± 2,0 | 3516 | 36,9 |
| hydrophiler Spray B | 4 | 12 | 0,8 | 1387 ± 630 $p < 0,001$ | 4,3 ± 1,6 $p < 0,05$ | 9534 $p< 0,001$ | 100 |
| lipophiler Spray B | 5 | 24 | 0,4 | 884 ± 693 | 7,9 ± 2,8 | 6990 ± 5158 | 73 |
| erfindungs- gemäßer Spray | 5 | 24 | 0,4 | 1774 ± 1272 $p = 0,006$ | 4,4 ± 1,4 $p < 0,05$ | 9488 ± 5303 u.s. | 100 |

Tab. 2 Rezepturzusammensetzung der Sprays

| Präparat | lipophiler Spray A | | lipophiler Spray B | | hydrophiler Spray A | hydrophiler Spray B | | erfindungsgemäßer Spray | |
|---|---|---|---|---|---|---|---|---|---|
| Quelle Nr. | 1 | | 1 | | 3 | Dictionnaire Vidale 1987 | | Zusammensetzung | |
| Inhalt | in 100 g | | in 100 g | | | in 100 ml | | in 100 g | |
| | GTN gelöst in | 0,9 | 0,7 | | hydrophiler wasser mischbarer | GTN 4% m/m in Ethanol | 34,0 ml | GTN 5% m/m in Ethanol | 14,56 |
| | Neutralöl | 27 | 15,0 | | Pumpspray | | | | |
| | Paraffin sub. | 12,4 | – | | | Ethanol 95 % v/v | 14,0 ml | 1,2-Propylen- glykol | 7,28 |
| | Ether | 2,2 | 1,8- | | | Diethylenglykol- | | – | |
| | Aroma | 0,5 | 0,4 | | | monoethlyehter | 1,0 ml | | |
| | Treibgas | 57,0 | 82,1 | | | Aroma | 1,0 ml | – | |
| | | | | | | Dichlordifluor- methan | 50,0 ml | Dichlordifluor- methan | 31,26 |
| | | | | | | | | Dichlortetra- fluorethan | 46,90 |

EP 0 461 505 B1

EP 0 461 505 B1

Tabelle 3

Wechselwirkung von Ventildichtungsmaterialien im erfindungsgemäßem Spray

## Absorption von Glyceroltrinitrat (GTN) in Dichtungsmaterial
[ mg GTN /1 g Dichtung]

| Dichtungsmaterial | Lagerbedingungen | | | |
| --- | --- | --- | --- | --- |
| | 20°C/<br>2 Wochen | 40°C/<br>2 Wochen | 20°C/<br>4 Wochen | 40°C/<br>4Wochen |
| Buna BA 170T<br>(aus Ventil WE 947) | 45,0 | 51,0 | 44,5 | 49,0 |
| Dichtung Nr. 400<br>(aus Ventil WE 999) | 53,2 | 61,1 | – | – |
| B 470 PA<br>(aus Ventil WE 1010) | 56,3 | 53,6 | – | – |
| RP 3-49-16<br>(aus Ventil WE 1008) | 44,0 | 49,0 | – | – |
| Neopren,schwarz<br>(aus Ventil WE 1007/1008) | 11,4 | 14,3 | 14,6 | 15,2 |
| Neopren, beige<br>(aus Ventil WE 1031) | 1,5-7,5 | 6,9-7,5 | 3,4 | 13,0 |
| Butyl FA 7500<br>(aus Ventil WE 1123,1124,<br>1125) | – | – | 0,05-0,07 | – |

EP 0 461 505 B1

Tabelle 4
Glyceroltrinitrat/Sprühstoß

| Lagerdauer [Monate] | Produkt Ch.-B. Dichtmaterial | | erfindungsgem. Spray 1 Butyl FA 7500 | | | | erfindungsgem. Spray 2 Neopren, kohlenstofffrei | | | |
|---|---|---|---|---|---|---|---|---|---|---|
| | Lagerdauer nach Herstellung und Primen [Wochen] | | Gehalt GTN [%]/1.-3. Sprühstoß | | | | Gehalt GTN [%]/ 1.-3. Sprühstoß | | | |
| | | | 1. | 2. | 3. | | 1. | 2. | 3. | |
| | 2,5 Lagerdauer zw. 2 Terminen [Wochen] | A<br>B | 74,5<br>75,7 | 83,2<br>87,8 | 89,1<br>91,6 | A<br>B<br>C | 76,0<br>78,2<br>71,8 | 93,3<br>92,0<br>91,3 | 99,5<br>97,5<br>96,4 | |
| 0,8 | 1 | A<br>B | 86,4<br>83,8 | 94,8<br>92,2 | 99,0<br>98,9 | A<br>B<br>C | 86,9<br>86,9<br>82,6 | 96,8<br>98,9<br>94,3 | 100,0<br>100,0<br>99,8 | |
| 1,5 | 3 | A<br>B | 85,2<br>83,1 | 92,0<br>96,7 | 100,7<br>98,0 | A<br>B<br>C | 76,9<br>79,4<br>80,9 | 93,4<br>92,0<br>90,2 | 96,7<br>96,3<br>99,2 | |
| 3,9 | 10 | A<br>B | 83,8<br>98,9 | 98,7<br>98,0 | 102,5<br>97,2 | A<br>B<br>C | 62,2<br>58,9<br>64,4 | 85,2<br>81,4<br>91,6 | 99,2<br>93,8<br>99,6 | |

Lagerbedingung 20 - 25°C/40-60% r.F.

Gehaltsangabe GTN [%]/Sprühstoß bezogen auf den Glyceroltrinitratgehalt/Sprühstoß, der nach mehrfachen Ventilabgaben als konstanter Wert bestimmt wurde (s.Tab.5)
A, B, C = Bezeichnung des Spraybehälters

EP 0 461 505 B1

Tabelle 5
Glyceroltrinitrat/Sprühstoß

| | Produkt Ch.-B. Dichtmaterial | erfindungsgem. Spray 1 Buna BA 170T | | | erfindungsgem. Spray 2 Dichtung Nr. 400 | | |
|---|---|---|---|---|---|---|---|
| Lagerdauer [Monate] | Lagerdauer nach Herstellung und Primen [Wochen] | Gehalt GTN [%]/ 1.-3. Sprühstoß | | | Gehalt GTN [%]/ 1.- 3. Sprühstoß | | |
| | | 1. | 2. | 3. | 1. | 2. | 3. |
| 1 | 4 | A 56,3 | 85,0 | 92,1 | A 69,7 | 77,4 | 81,2 |
| | | B 55,6 | 80,9 | 94,1 | B 72,0 | 82,0 | 86,2 |
| | Lagerdauer zw. 2 Terminen [Wochen] | | | | C 69,3 | – | 86,8 |
| 1,25 | 1 | A 54,0 | 83,5 | 94,2 | A 65,0 | 75,2 | 85,3 |
| | | B 59,5 | 82,5 | 98,5 | B 51,1 | 69,1 | 82,3 |
| 2 | 3 | A 48,8 | 81,8 | 94,1 | | | |
| | | B 48,8 | 87,7 | 104,9 | | | |
| 4,5 | 10 | A 63,5 | 87,7 | 92,7 | Dichtung Nr. 400: Ein GTN-Gehalt von 100 %/ | | |
| | | B 51,8 | – | 95,3 | Sprühstoß wird erst ab dem ≈ 15. Sprühstoß erreicht. | | |

Lagerbedingung 20 - 25°C/40-60% r.F.

Gehaltsangabe GTN [%]/Sprühstoß bezogen auf den Glyceroltrinitratgehalt/Sprühstoß, der nach mehrfachen Ventilabgaben als konstanter Wert bestimmt wurde.
A, B, C = Bezeichnung des Spraybehälters

EP 0 461 505 B1

**Patentansprüche**

**Patentansprüche für folgende Vertragsstaaten : AT, BE, CH, DE, DK, FR, GB, IT, LI, LU, NL, SE**

1. Nitroglycerin-Spray in der Zusammensetzung aus Nitroglycerin, Ethanol, 1,2-Propylenglykol und Treibgas, dadurch gekennzeichnet, daß die Zusammensetzung aus 0,73 Gew.-% Nitroglycerin, 13,83 Gew.-% Ethanol, 7,28 Gew.-% 1,2-Propylenglykol, 31,26 Gew.-% Dichlordifluormethan und 46,90 Gew.-% Dichlortetrafluorethan in ständigem Kontakt mit Behältnisdichtmaterial steht, dessen Absorptionswert für Nitroglycerin geringer als 10 mg/1 g Dichtmaterial ist.

2. Nitroglycerin-Spray nach Anspruch 1, dadurch gekennzeichnet, daß das Behältnisdichtmaterial Butyl FA 7500 ist.

3. Nitroglycerin-Spray nach Anspruch 1, dadurch gekennzeichnet, daß das Behältnisdichtmaterial ein kohlenstofffreies Neopren ist.

4. Verwendung von Behältnisdichtmaterial mit Absorptionswerten für Nitroglycerin unter 10 mg/1 g Dichtmaterial in treibgashaltigen Nitroglycerin-Spravvorrichtungen.

**Patentansprüche für folgende Vertragsstaaten : ES, GR**

1. Verfahren zur Herstellung eines Nitroglycerin-Sprays in der Zusammensetzung aus Nitroglycerin, Ethanol, 1,2-Propylenglykol und Treibgas, dadurch gekennzeichnet, daß eine homogene, einphasige Lösung, bestehend aus 0,73 Gew.-% Nitroglycerin, 13,83 Gew.-% Ethanol, 7,28 Gew.-% 1,2-Propylenglykol in Aerosoldosen oder Aerosolflaschen gefüllt wird, ein geeignetes mit Dichtmaterial versehenes Dosierventil, dessen Absorptionswert für Glyceroltrinitrat geringer als 10 mg/1 g Dichtmaterial beträgt, aufgecrimpt wird und mit 31,26 Gew.-% Dichlordifluormethan und 46,90 Gew.-% Dichlortetrafluorethan begast wird.

2. Verfahren zur Herstellung eines Nitroglycerin-Sprays nach Anspruch 1, dadurch gekennzeichnet, daß als Behältnisdichtmaterial Butyl FA 7500 verwendet wird.

3. Verfahren zur Herstellung eines Nitroglycerin-Sprays nach Anspruch 1, dadurch gekennzeichnet, daß als Behältnisdichtmaterial kohlenstofffreies Neopren verwendet wird.

4. Verfahren zur Verwendung von Behältnisdichtmaterial mit Absorptionswerten für Nitroglycerin unter 10 mg/1 g Dichtmaterial in treibgashaltigen Nitroglycerin-Sprayvorrichtungen.

**Claims**

**Claims for the following Contracting States : AT, BE, CH, DE, DK, FR, GB, IT, LI, LU, NL, SE**

1. Nitroglycerin spray comprising nitroglycerin, ethanol, 1,2-propyleneglykol and propellant gas characterized in that the composition consisting of 0,73% by weight of nitroglycerin, 13,83% by weight of ethanol, 7,28% by weight of 1,2-propyleneglycol, 31,26% by weight of dichlorodifluoromethane and 46,90% by weight of dichlorotetrafluoroethane is in continuous contact with container sealing material which has a nitroglycerin absorption value less than 10 mg/1 g of sealing material.

2. Nitroglycerin spray according to claim 1 characterized in that the container sealing material is Butyl FA 7500.

3. Nitroglycerin spray according to claim 1 characterized in that the container sealing material is neoprene free of carbon.

4. The use of container sealing material which has nitroglycerin absorption values less than 10 mg/1 g of sealing material in aerosol spray propellant gas containers containing nitroglycerin.

12

**Claims for the following Contracting States : ES, GR**

1. Process for preparing a nitroglycerin spray composed of nitroglycerin, ethanol, 1,2-propylene glycol and propellant gas, characterized in that a homogenous, monophasic solution, consisting of 0,73% by weight of nitroglycerin, 13,83% by weight of ethanol, 7,28% by weight of 1,2-propylene glycol is filled into aerosol cans or aerosol bottles, crimping on a suitable metering valve whose absorption value for glycerol trinitrate is less than 10 mg/1 g of sealing material, and pressurising with a made-up mixture of dichlorodifluormethane and dichlorotetrafluoroethane.

2. Process for preparing a nitroglycerin spray according to claim 1, characterized in that Butyl FA 7500 is used as the container sealing material.

3. Process for preparing a nitroglycerin spray according to claim 1, characterized in that carbon-free Neoprene is used as the container sealing material.

4. Process for the use of container sealing material with absorption values for nitroglycerin less than 10 mg/1 g of sealing material in propellant gas containing nitroglycerin-spray-devices.

**Revendications**

**Revendications pour les Etats contractants suivants : AT, BE, CH, DE, DK, FR, GB, IT, LI, LU, NL, SE**

1. Préparation de spray contenant de la nitroglycérine composée de la nitroglycérine, éthanol, 1,2-propylèneglycol et du gaz propulseur, charactérisée en ce que la préparation contient de 0,73% en poids de nitroglycérine, de 13,83% en poids d'éthanol, de 7,28% en poids de 1,2-propylèneglycol, de 31,26 en poids de dichlordifluorméthane et de 46,90% en poids de dichlortetrafluoréthane et étant contactée permanente avec le matériau d'étanchéité du conteneur duquel valeur d'absorption pour la nitroglycérine fait moins que 10 mg/1 g du matériau d'étanchéité.

2. Préparation de spray contenant de la nitroglycérine selon la revendication 1, charactérisée en ce que le marériau d'étanchéité du conteneur est Butyl FA 7500.

3. Préparation de spray contenant de la nitroglycérine selon la revendication 1, charactérisée en ce que le matériau d'étanchéité du conteneur est néoprène libre du carbone.

4. Utilisation du matériau d'étanchéité du conteneur avec des valeurs d'absorption pour de la nitroglycérine moins que 10 mg/1 g matériau d'étanchéité dans des dispositifs de spray contenant de la nitroglycérine et du gaz propulseur.

**Revendications pour les Etats contractants suivants : ES, GR**

1. Procédé de préparation d'une préparation de spray contenant de la nitroglycérine composée de la nitroglycérine, éthanol,1,2-propylèneglycol et du gaz propulseur, charactérisé en ce qu'une solution homogène monophasique contenant de 0,73% en poids de nitroglycérine, de 13,83% en poids d'éthanol, de 7,28% en poids de 1,2-propylèneglycol est introduite dans des boîtes pour aérosol ou dans des flacons de verre pour aérosol,une soupape de dosage appropriée pourvue du matériau d'étanchéité, duquel valeur d'absorption étant moins que 10 mg/1 g matériau d'étanchéité est serrée et enfin gazée avec de 31,26% en poids de dichlordifluorméthane et de 46,90% en poids de dichlortetrafluoréthane.

2. Procédé de préparation d'une préparation de spray contenant de la nitroglycérine selon la revendication 1, charactérisé en ce qu'on utilise Butyl FA 7500 comme matériau d'étanchéité du conteneur.

3. Procédé de préparation d'une préparation de spray contenant de la nitroglycérine selon revendication 1, charactérisé en ce qu'on utilise néoprène libre du carbone comme matériau d'étanchéité du conteneur.

4. Procédé d'utilisation du matériau d'étanchéité du conteneur avec des valeurs d'absorption pour de la nitroglycérine moins que 10 mg/1 g matériau d'étanchéité dans des dispositifs de spray contenant de la nitroglycérine et du gaz propulseur.